# EUROPEAN PATENT APPLICATION

(11) **EP 4 538 351 A1**
(43) Date of publication of application: **16.04.2025**
(21) Application number: 23823837.2
(22) Date of filing: 09.06.2023
(51) Int. Cl.: C10G 2/00, C01B 3/38, C01B 32/40, C10G 1/10, C10G 35/04, C10G 50/00

(54) **LIQUID HYDROCARBON PRODUCTION METHOD AND LIQUID HYDROCARBON PRODUCTION DEVICE**

(30) Priority: 13.06.2022 JP 2022095204
(71) Applicant: INPEX Corporation, Tokyo 107-6332 (JP); National University Corporation University Of Toyama, Toyama-shi, Toyama 930-8555 (JP)
(72) Inventor: SANO, Yosuke, Tokyo 107-6332 (JP); KURIMURA, Hideki, Tokyo 107-6332 (JP); AOYAMA, Takuya, Tokyo 107-6332 (JP); YONAMINE, Wataru, Tokyo 107-6332 (JP); TSUBAKI, Noritatsu, Toyama-shi, Toyama 930-8555 (JP)
(74) Representative: Cabinet Beaumont
(86) International application number: PCT/JP2023/021443
(87) International publication number: WO 2023/243547

(57) **Abstract**

Provided are a liquid hydrocarbon producing method and a liquid hydrocarbon producing apparatus, which are for producing liquid hydrocarbons at a high efficiency by suppressing the addition of oxygen to carbon.

In the liquid hydrocarbon producing method, a carbon compound is pyrolyzed using a metal oxide as a heat medium to generate a first gas containing H₂, CH₄, and light olefins; solid carbon formed as a by-product on the surface of the metal oxide during the pyrolysis is partially oxidized in a CO₂ atmosphere to generate a second gas containing CO; steam is supplied to the second gas and a light saturated hydrocarbon gas and a steam reforming reaction and a water gas shift reaction are conducted to generate a third gas containing CO and H₂; and a liquid hydrocarbon is produced using the first gas and the third gas as raw materials.

## Description

### Technical Field

The present invention relates to a liquid hydrocarbon producing method and a liquid hydrocarbon producing apparatus, which are for producing liquid hydrocarbons from carbon compounds such as waste plastics.

### Background Art

From the perspective of environmental issues, the arrival of a net zero carbon society is eagerly awaited, and the concept of carbon recycling has been proposed as one of the methods to achieve this. Effective use of waste materials is one of the themes in this concept, but there are quite a few problems. For example, waste plastics are burned and power is generated using the thermal energy obtained during the combustion. However, the proportion of energy that can be recovered is limited, and further there are problems in that a large amount of carbon dioxide (CO₂) is emitted. As one method to solve this problem, technologies have been developed for obtaining an energy source with a low environmental impact by gasifying waste materials such as waste plastics and using the obtained gas as a raw material to produce useful liquid hydrocarbons such as liquid fuels. Patent Literature 1 discloses a technology for producing liquid hydrocarbons from waste materials.

### Citation List

### Patent Literature:

Patent Literature 1: Japanese Published Patent Publication No. 2018-525510 Summary

### Technical Problems

In the technology described in Patent Literature 1, carbon compounds contained in waste materials are decomposed into carbon monoxide (CO) and hydrogen gas (H₂) during gasification. This increases the amount of oxygen that is required to be removed when liquid hydrocarbons are produced from the gas as a raw material. In order to remove oxygen, H₂ is consumed to form water (H₂O), and thus hydrogen contained in the carbon compounds is consumed. The consumption of hydrogen decreases the yield of liquid hydrocarbons formed using carbon compounds contained in waste materials as a raw material.

The present invention has been made in view of the above circumstances, and an object thereof is to provide a liquid hydrocarbon producing method and a liquid hydrocarbon producing apparatus, which are for producing liquid hydrocarbons at a high efficiency by suppressing the bonding of oxygen to carbon.

### Solution to Problems

A liquid hydrocarbon producing method according to one aspect of the present invention is characterized by comprising: pyrolyzing a carbon compound using a metal oxide as a heat medium to generate a first gas containing H₂, CH₄, and light olefins; partially oxidizing solid carbon formed as a by-product on a surface of the metal oxide during the pyrolysis in a CO₂ atmosphere to generate a second gas containing CO; supplying steam to the second gas and a light saturated hydrocarbon gas and conducting a steam reforming reaction and a water gas shift reaction to generate a third gas containing CO and H₂; and producing a liquid hydrocarbon using the first gas and the third gas as raw materials.

In the liquid hydrocarbon producing method according one aspect of the present invention, it is characterized in that a liquid hydrocarbon having a carbon chain length equal to or more than a predetermined length is produced by conducting an oligomer reaction and a Fischer-Tropsch reaction using the first gas and the third gas.

In the liquid hydrocarbon producing method according one aspect of the present invention, it is characterized in that the light saturated hydrocarbon gas is generated as a by-product when the oligomer reaction or the Fischer-Tropsch reaction is conducted, and the steam reforming reaction is conducted using the generated light saturated hydrocarbon gas to generate H₂.

In the liquid hydrocarbon producing method according one aspect of the present invention, it is characterized in that the solid carbon is reacted with oxygen in the metal oxide in a CO₂ atmosphere to conduct partial oxidation of the solid carbon, and the metal oxide that is in a reduced state by the partial oxidation is oxidized with oxygen in air to generate heat.

In the liquid hydrocarbon producing method according one aspect of the present invention, it is characterized in that naphtha and kerosene are formed as the liquid hydrocarbon in the oligomer reaction and the Fischer-Tropsch reaction, and a reforming reaction is conducted on the formed naphtha to form BTX.

In the liquid hydrocarbon producing method according one aspect of the present invention, it is characterized in that H₂ generated by the reforming reaction is used to conduct the oligomer reaction and the Fischer-Tropsch reaction.

A liquid hydrocarbon producing apparatus according to one aspect of the present invention is characterized by comprising: a pyrolysis furnace for pyrolyzing a carbon compound using a metal oxide as a heat medium to generate a first gas containing H₂, CH₄, and light olefins; a partial oxidation furnace for partially oxidizing solid carbon formed as a by-product on a surface of the metal oxide during the pyrolysis in a CO₂ atmosphere to generate a second gas containing CO; a first reactor for supplying steam to the second gas and a light saturated hydrocarbon gas and conducting a steam reforming reaction and a water gas shift reaction to generate a third gas containing CO and H₂; and a second reactor for conducting an oligomer reaction of the first gas and a Fischer-Tropsch reaction of the third gas to produce a liquid hydrocarbon having a carbon chain length equal to or more than a predetermined length.

In an aspect of the present invention, carbon compounds such as waste plastics are pyrolyzed using a metal oxide as a heat medium to generate a first gas containing H₂, CH₄ and light olefins. The solid carbon formed by the pyrolysis is partially oxidized in a CO₂ atmosphere to generate a second gas containing CO. Steam is supplied to the second gas and a light saturated hydrocarbon gas and a steam reforming reaction and a water gas shift reaction are conducted to generate a third gas containing CO and H₂. Liquid hydrocarbons are produced using the first gas and the third gas as raw materials. During the pyrolysis of carbon compounds, the carbon compounds are not completely decomposed into CO and H₂, so that the energy required for the pyrolysis decreases. Since the first gas containing CH₄ and light olefins is used, the amount of oxygen atoms bonded to carbon atoms decreases compared to a conventional method in which carbon compounds are decomposed to CO and H₂. The amount of hydrogen atoms consumed to remove the oxygen atoms decreases, and the yield of liquid hydrocarbons produced is improved. As the pyrolysis of carbon compounds is conducted using a metal oxide as a heat medium, the amount of nitrogen mixed into the raw material for liquid hydrocarbons is cut down compared to a conventional method in which carbon compounds are burned. By converting a part of the light saturated hydrocarbon gas into CO and H₂ by the steam reforming reaction and a part of the second gas into CO₂ and H₂ by the water gas shift reaction, the ratio of light hydrocarbons, CO and H₂, which are the raw materials for liquid hydrocarbons can be adjusted.

In an aspect of the present invention, liquid hydrocarbons consisting of hydrocarbons having a long carbon chain are produced by an oligomer reaction and a Fischer-Tropsch reaction using the first gas and the third gas. Liquid hydrocarbons are produced at a high yield by an oligomer reaction using the first gas containing CH₄ and light olefins. Liquid hydrocarbons are efficiently produced by a Fischer-Tropsch reaction using the third gas in which the ratio of CO and H₂ has been adjusted by the steam reforming reaction and the water gas shift reaction.

In an aspect of the present invention, a steam reforming reaction is conducted using light saturated hydrocarbon gases such as CH₄ and C₂H₆ generated as by-products of the oligomer reaction or the Fischer-Tropsch reaction. Carbon that has not been contained in the desired liquid hydrocarbons at the stage where the oligomer reaction or Fischer-Tropsch reaction has been conducted undergoes the steam reforming reaction and is used again in the Fischer-Tropsch reaction. As the carbon that has not been contained in the desired liquid hydrocarbons is repeatedly used in the Fischer-Tropsch reaction, the yield of forming the desired liquid hydrocarbons from carbon derived from carbon compounds can be improved.

In an aspect of the present invention, CO is generated from solid carbon by conducting partial oxidation in which solid carbon is reacted with oxygen in a metal oxide in a CO₂ atmosphere. As a result of the reaction of oxygen in a metal oxide with solid carbon, the metal oxide becomes in a reduced state. As the metal oxide in a reduced state is oxidized with oxygen in the air, the metal oxide generates heat. The heated metal oxide is used as a heat medium for the pyrolysis of carbon compounds. Since solid carbon does not come into direct contact with the air, mixing of nitrogen into the raw materials for liquid hydrocarbons can be suppressed.

In an aspect of the present invention, naphtha and kerosene are formed as liquid hydrocarbons in the oligomer reaction and the Fischer-Tropsch reaction. BTX is formed by conducting a reforming reaction on naphtha. Kerosene can be used as a liquid fuel. BTX is used as an industrial raw material. BTX can be obtained as a by-product during the production of liquid fuels.

In an aspect of the present invention, H₂ generated by the reforming reaction of naphtha is used to conduct the oligomer reaction and the Fischer-Tropsch reaction. Hydrogen that has not been contained in the liquid hydrocarbons is used again in the oligomer reaction and the Fischer-Tropsch reaction. As hydrogen that has not been contained in the liquid hydrocarbons is repeatedly used in the oligomer reaction and the Fischer-Tropsch reaction, the yield of forming liquid hydrocarbons from hydrogen derived from carbon compounds such as waste plastics can be improved.

### Advantageous Effects of Invention

According to the present invention, the energy required for the pyrolysis of carbon compounds decreases, and the yield of liquid hydrocarbons produced is improved. For this reason, the present invention exhibits excellent effects such that liquid hydrocarbons can be produced at a high efficiency.

### Brief Description of Drawings

FIG. 1 is a conceptual diagram illustrating an outline of a liquid hydrocarbon producing method.
FIG. 2 is a block diagram of an example of a liquid hydrocarbon producing apparatus.
FIG. 3 is a block diagram illustrating a configuration example of an experimental apparatus for conducting an experiment to form hydrocarbons by an oligomer reaction and a Fischer-Tropsch reaction.
FIG. 4 is a graph illustrating the amount of hydrocarbons formed in an experiment.

### Description of Embodiments

Hereinafter, the present invention will be specifically described with reference to the drawings illustrating embodiments thereof.

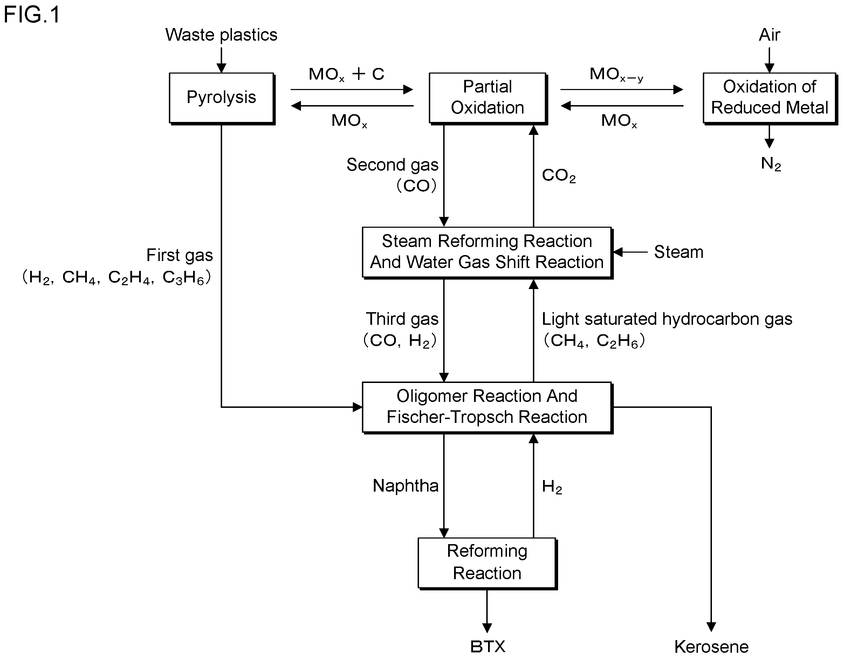

In the present embodiment, waste plastics, which are synthetic resins contained in waste materials, are pyrolyzed, and kerosene is formed by an oligomer reaction and a Fischer-Tropsch reaction using the gas generated by the pyrolysis. Waste plastics correspond to carbon compounds. FIG. 1 is a conceptual diagram illustrating the outline of the liquid hydrocarbon producing method. In this embodiment, waste plastics are pyrolyzed using a metal oxide as a heat medium. In FIG. 1, the metal oxide is represented by MOₓ, where x is a natural number. The metal oxide is, for example, iron oxide. The temperature during pyrolysis is 500°C or more. The components of waste plastics are, for example, polypropylene (PP), polystyrene (PS) or polyethylene (PE). By the pyrolysis of waste plastics, the first gas mainly composed of hydrogen gas (H₂), methane (CH₄) and light olefins is generated. Here, the term "light" means that the carbon chain length is short and the substance is volatile. Light olefins are volatile olefins and are, for example, olefins in which the number of carbon atoms contained in the carbon chain is less than six, such as C₂H₄ and C₃H₆. An example of the pyrolysis of waste plastics is expressed by the following Formula (1).

PP + PS + PE → H₂ + CH₄ + C₂H₄ + C₃H₆ ... (1)

During the pyrolysis of waste plastics, solid carbon is formed as a by-product, and the formed solid carbon is attached to the metal oxide. In FIG. 1, solid carbon is represented by C. CO₂ is introduced into the metal oxide to which solid carbon is attached, and the solid carbon is partially oxidized in a CO₂ atmosphere. The solid carbon reacts with oxygen in the metal oxide to generate carbon monoxide (CO). The chemical reaction formula for the partial oxidation of solid carbon is expressed by the following Formula (2), where y is a natural number.

yC + MOₓ → yCO + MO_{x-y} ... (2)

In a situation where there are excess of solid carbon and excess of heat, the solid carbon reacts with CO₂ to generate CO. CO₂ can be converted into CO, which is a raw material for liquid hydrocarbons. The chemical formula for the reaction of solid carbon with CO₂ is expressed by the following Formula (3).

C + CO₂ → 2CO... (3)

A second gas mainly composed of CO is generated by the partial oxidation of solid carbon. As the oxygen in the metal oxide reacts with the solid carbon, the metal oxide is reduced. The reduced metal oxide is represented by MO_{x-y}. The oxidation of the reduced metal oxide is conducted by bringing the reduced metal oxide into contact with the air. The chemical reaction formula for the oxidation of the reduced metal oxide is expressed by the following Formula (4).

MO_{x-y} + 0.5O₂ → MOₓ ... (4)

Since oxygen (O₂) in the air is consumed by the oxidation of the reduced metal oxide, nitrogen (N₂) is generated. N₂ is discharged. Heat is generated by the oxidation of the reduced metal oxide, and the metal oxide is heated. The oxidized metal oxide in a heated state is utilized as a heat source for the partial oxidation of solid carbon. Thereafter, the metal oxide in a heated state is further utilized as a heat source for the pyrolysis of waste plastics. By utilizing the heat generated by the oxidation of the reduced metal oxide, energy can be efficiently utilized to form liquid hydrocarbons.

A steam reforming reaction is conducted using steam and a light saturated hydrocarbon gas, which is a by-product of the Fischer-Tropsch reaction described later. The light saturated hydrocarbon gas is a gas composed of light saturated hydrocarbons. Light saturated hydrocarbons are volatile saturated hydrocarbons and, for example, saturated hydrocarbons having six or less carbon atoms in the molecule, such as CH₄ and C₂H₆. The chemical reaction formula for an example of the steam reforming reaction is expressed by the following Formula (5). By the steam reforming reaction, CO and H₂ are generated from light saturated hydrocarbons.

CH₄+ H₂O → CO + 3H₂ ... (5)

A water gas shift reaction is conducted using the CO generated by the steam reforming reaction and the CO contained in the second gas and steam. The chemical reaction formula for an example of the water gas shift reaction is expressed by the following Formula (6).

CO + H₂O → CO₂ + H₂ ... (6)

By the water gas shift reaction, CO₂ and H₂ are generated. The CO₂ and H₂ are separated from each other by a separation process such as PSA (pressure swing adsorption). The separated CO₂ is utilized in the partial oxidation of solid carbon. A third gas is generated which is composed of CO and H₂ generated by the steam reforming reaction and the water gas shift reaction and a part of CO contained in the second gas. The third gas is mainly composed of CO and H₂. The oligomer reaction and the Fischer-Tropsch reaction are conducted using the first gas generated by the pyrolysis of waste plastics and the third gas. The chemical reaction formula for an example of the oligomer reaction is expressed by the following Formula (7), and the chemical reaction formula for an example of the Fischer-Tropsch reaction is expressed by the following Formula (8). n and m in Formula (7) are natural numbers of 2 or more, and n in Formula (8) is a natural number of 1 or more.

CₙH₂ₙ + CₘH₂ₘ + H₂ → C₍ₙ₊ₘ₎H₍₂ₙ₊₂ₘ₊₂₎ ... (7)

nCO + 2(n+1)H₂ → CₙH₂ₙ₊₂ + nH₂O ... (8)

By the oligomer reaction and the Fischer-Tropsch reaction, hydrocarbons having a carbon chain length equal to or more than a predetermined length are formed using light hydrocarbons including light olefins, CO, and H₂ as raw materials. The predetermined length of carbon chain is the length at which the hydrocarbon becomes liquid. For example, the carbon chain length equal to or more than the predetermined length is a length in which the number of carbon atoms contained in the carbon chain is six or more. As a hydrocarbon having a carbon chain length equal to or more than the predetermined length is formed, a liquid hydrocarbon is formed. Among liquid hydrocarbons, relatively light ones become naphtha, and relatively heavy ones become kerosene. In addition, a light saturated hydrocarbon gas, which is a by-product, is generated. A light saturated hydrocarbon gas is a light hydrocarbon having a carbon chain length less than the predetermined length.

The light saturated hydrocarbon gas is used in the steam reforming reaction. Thus, carbon that has not been contained in the liquid hydrocarbons undergoes the steam reforming reaction and is used again in the Fischer-Tropsch reaction. As the carbon that has not been contained in the liquid hydrocarbons is repeatedly used in the Fischer-Tropsch reaction, the yield of forming liquid hydrocarbons from carbon derived from waste plastics can be improved.

In order to properly conduct the oligomer reaction and the Fischer-Tropsch reaction, it is desirable that the ratio of the amounts of light hydrocarbons, CO, and H₂ is within a predetermined range. For example, for an efficient Fischer-Tropsch reaction, the ratio of H₂ to CO is desirably 3 or more. By converting a part of the light saturated hydrocarbon gas into CO and H₂ by the steam reforming reaction and a part of the CO into CO₂ and H₂ by the water gas shift reaction, the ratio of light hydrocarbons, CO and H₂, which are the raw materials for hydrocarbons, can be adjusted so that the oligomer reaction and the Fischer-Tropsch reaction are properly conducted.

A reforming reaction is conducted on naphtha formed by the oligomer reaction and the Fischer-Tropsch reaction. The reforming reaction is a reaction for dehydrogenating saturated hydrocarbons contained in naphtha. The chemical reaction formula for an example of the reforming reaction is expressed by the following Formula (9).

C₇H₁₆ → C₇H₈ + 4H₂ ... (9)

Formula (9) is the chemical reaction formula for the reaction to convert heptane contained in naphtha into toluene. BTX is formed by conducting a reforming reaction on naphtha. BTX is a general term for benzene, toluene and xylene. The BTX is recovered. The BTX is used as an industrial raw material. For example, the BTX is used as a raw material for synthetic resins.

H₂ generated by the reforming reaction is used in the oligomer reaction and the Fischer-Tropsch reaction. Thus, the hydrogen that has not been contained in the liquid hydrocarbons is used again in the oligomer reaction and the Fischer-Tropsch reaction. As the hydrogen that has not been contained in the liquid hydrocarbons is repeatedly used in the oligomer reaction and the Fischer-Tropsch reaction, the yield of forming liquid hydrocarbons from hydrogen derived from waste plastics can be improved. By utilizing H₂ generated by the reforming reaction, it is not necessary to supply H₂ from the outside to produce liquid hydrocarbons. Since liquid hydrocarbons can be produced without supplying H₂, which requires a large amount of energy to be produced, from the outside, the energy required to form liquid hydrocarbons decreases.

Kerosene formed by the oligomer reaction and the Fischer-Tropsch reaction is recovered. The kerosene may be used as a liquid fuel. The kerosene may be used singly as a liquid fuel, or may be mixed with other liquid hydrocarbons and used as a liquid fuel. In this manner, a liquid fuel such as aviation fuel is produced.

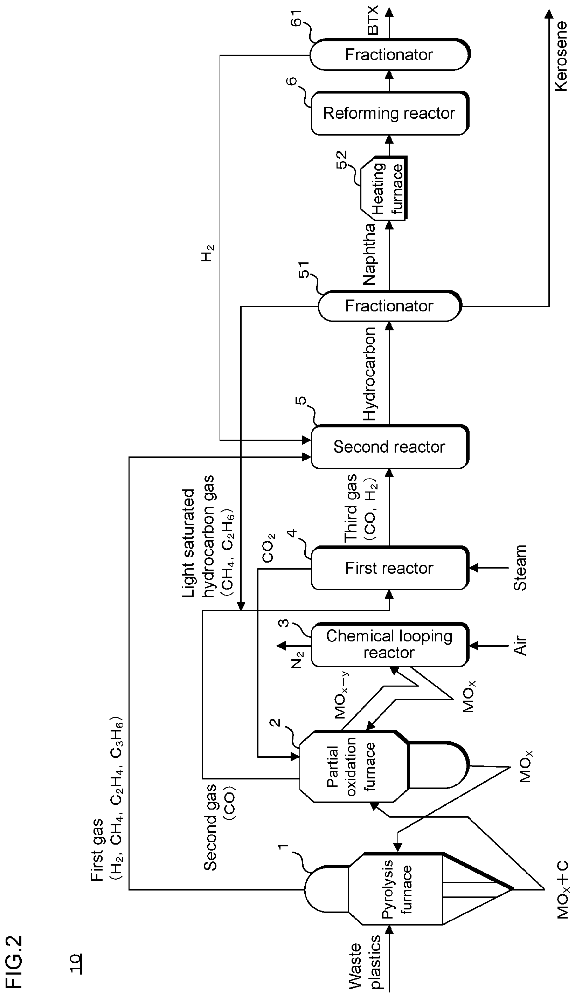

FIG. 2 is a block diagram of an example of a liquid hydrocarbon producing apparatus 10. The liquid hydrocarbon producing apparatus 10 is an apparatus that carries out the outline of the liquid hydrocarbon producing method. The liquid hydrocarbon producing apparatus 10 includes a pyrolysis furnace 1, a partial oxidation furnace 2, and a chemical looping reactor 3. The pyrolysis furnace 1 pyrolyzes waste plastics using a metal oxide as a heat medium to generate a first gas. The partial oxidation furnace 2 partially oxidizes solid carbon attached to the metal oxide in a CO₂ atmosphere to generate a second gas. The chemical looping reactor 3 conducts oxidation of the metal oxide.

The pyrolysis furnace 1 is composed of a fluidized bed reactor. The pyrolysis furnace 1 and the partial oxidation furnace 2 are connected to each other via a conveying path for conveying a metal oxide from the pyrolysis furnace 1 to the partial oxidation furnace 2 and a conveying path for conveying a metal oxide from the partial oxidation furnace 2 to the pyrolysis furnace 1. The pyrolysis furnace 1 is equipped with a mechanism for charging waste plastics, a mechanism for charging a metal oxide conveyed from the partial oxidation furnace 2, a mechanism for discharging the metal oxide, and a mechanism for discharging the first gas. A heated metal oxide and waste plastics are charged into the pyrolysis furnace 1, and the waste plastics and metal oxide are agitated by the fluidized bed. The pyrolysis furnace 1 may be equipped with a mechanism for heating the inside thereof or a mechanism for heating the metal oxide.

The waste plastics are pyrolyzed by the heat of the metal oxide, as presented in Formula (1). As the waste plastics are pyrolyzed, the first gas is generated and solid carbon formed as a by-product is attached to the metal oxide. The first gas is discharged from the pyrolysis furnace 1 and the metal oxide to which solid carbon is attached is conveyed from the pyrolysis furnace 1 to the partial oxidation furnace 2.

The partial oxidation furnace 2 is composed of a reactor. The partial oxidation furnace 2 and the chemical looping reactor 3 are connected to each other via a conveying path for conveying a metal oxide from the partial oxidation furnace 2 to the chemical looping reactor 3 and a conveying path for conveying a metal oxide from the chemical looping reactor 3 to the partial oxidation furnace 2. The partial oxidation furnace 2 is equipped with a mechanism for charging the metal oxide to which solid carbon is attached, conveyed from the pyrolysis furnace 1 and a mechanism for charging the metal oxide conveyed from the chemical looping reactor 3. The partial oxidation furnace 2 is connected to a first reactor 4 described later via a pipe. From the first reactor 4, CO₂ flows through the pipe to the partial oxidation furnace 2. The partial oxidation furnace 2 is equipped with a mechanism for introducing CO₂ from the first reactor 4.

Into the partial oxidation furnace 2, the metal oxide to which solid carbon is attached from the pyrolysis furnace 1 and the metal oxide from the chemical looping reactor 3 are charged, and CO₂ from the first reactor 4 is introduced. The metal oxide from the chemical looping reactor 3 has been heated. The partial oxidation furnace 2 may be equipped with a mechanism for heating the inside thereof or a mechanism for heating the metal oxide. As presented in Formula (2), the solid carbon attached to the metal oxide from the pyrolysis furnace 1 is partially oxidized to generate CO in the CO₂ atmosphere by the heat of the metal oxide from the chemical looping reactor 3. In addition, as presented in Formula (3), solid carbon reacts with CO₂ to generate CO. In this manner, a second gas mainly composed of CO is generated and the metal oxide from the pyrolysis furnace 1 is reduced.

The partial oxidation furnace 2 is equipped with a mechanism for discharging the second gas, a mechanism for discharging the metal oxide from the pyrolysis furnace 1, and a mechanism for discharging the metal oxide from the chemical looping reactor 3. The generated second gas is discharged from the partial oxidation furnace 2. The metal oxide from the chemical looping reactor 3 is conveyed from the partial oxidation furnace 2 to the pyrolysis furnace 1. The metal oxide from the pyrolysis furnace 1 is reduced and conveyed from the partial oxidation furnace 2 to the chemical looping reactor 3.

The chemical looping reactor 3 is composed of a reactor. The chemical looping reactor 3 is equipped with a mechanism for introducing air from the outside, a mechanism for charging the reduced metal oxide conveyed from the partial oxidation furnace 2, a mechanism for discharging the metal oxide, and a mechanism for discharging N₂. Into the chemical looping reactor 3, the reduced metal oxide from the partial oxidation furnace 2 is charged, and air is introduced, and the reduced metal oxide is oxidized as presented in Formula (4). The reduced metal oxide reacts with oxygen in the air and the oxygen is bonded to the metal oxide again. Oxygen in the air is consumed and N₂ is generated. In addition, heat is generated by the oxidation of the metal oxide, and the metal oxide is heated. The generated N₂ is discharged from the chemical looping reactor 3. The heated metal oxide is conveyed from the chemical looping reactor 3 to the partial oxidation furnace 2.

The liquid hydrocarbon producing apparatus 10 further includes a first reactor 4, a second reactor 5, a fractionator 51, a heating furnace 52, a reforming reactor 6, and a fractionator 61. The first reactor 4 is a reactor for conducting the steam reforming reaction and the water gas shift reaction. For example, the first reactor 4 is composed of a fixed bed reactor and contains catalysts for the steam reforming reaction and the water gas shift reaction therein. For example, a large number of catalyst carriers carrying catalysts on their surfaces are packed inside the first reactor 4.

The first reactor 4 is connected to the partial oxidation furnace 2 via a pipe separate from the pipe through which CO₂ passes. The second gas discharged from the partial oxidation furnace 2 flows through the pipe to the first reactor 4. In addition, the first reactor 4 is connected to the fractionator 51 via a pipe. From the fractionator 51, a light saturated hydrocarbon gas, which is a by-product of the oligomer reaction and the Fischer-Tropsch reaction, flows through the pipe to the first reactor 4. For example, the light saturated hydrocarbon gas includes CH₄ and C₂H₆. The first reactor 4 is equipped with a mechanism for introducing the second gas from the partial oxidation furnace 2, a mechanism for introducing the light saturated hydrocarbon gas from the fractionator 51, and a mechanism for introducing steam from the outside.

In the first reactor 4, the steam reforming reaction and the water gas shift reaction take place. In the steam reforming reaction, light saturated hydrocarbons react with H₂O to generate CO and H₂ as exemplified in Formula (5). In the water gas shift reaction, CO reacts with H₂O to generate CO₂ and H₂ as exemplified in Formula (6). The first reactor 4 is equipped with a mechanism for separating CO₂ by a separation process such as PSA. As CO₂ is separated from the generated gas, a third gas mainly composed of CO and H₂ is generated. The third gas also contains CO contained in the second gas.

The first reactor 4 is connected to the second reactor 5 via a pipe. The first reactor 4 is equipped with a mechanism for discharging the generated third gas. The generated third gas is discharged from the first reactor 4 and flows through the pipe to the second reactor 5. The first reactor 4 is equipped with a mechanism for discharging the separated CO₂. The discharged CO₂ is introduced into the partial oxidation furnace 2 through the pipe. The reactor for conducting the steam reforming reaction and the reactor for conducting the water gas shift reaction may be separated from each other. The first reactor 4 may be composed of the reactor for the steam reforming reaction and the reactor for the water gas shift reaction, which are separated from each other.

The second reactor 5 is a reactor for conducting the oligomer reaction and the Fischer-Tropsch reaction. For example, the second reactor 5 is composed of a fixed bed reactor and contains catalysts for the oligomer reaction and the Fischer-Tropsch reaction therein. For example, a large number of catalyst carriers carrying catalysts on their surfaces are packed inside the second reactor 5. The pyrolysis furnace 1 and the second reactor 5 are connected to each other via a pipe. The first gas discharged from the pyrolysis furnace 1 flows through the pipe to the second reactor 5. The second reactor 5 is connected to the fractionator 61 via a pipe. From the fractionator 61, the gas containing H₂ flows through the pipe to the second reactor 5. The second reactor 5 is equipped with a mechanism for introducing the first gas from the pyrolysis furnace 1, a mechanism for introducing the third gas from the first reactor 4, and a mechanism for introducing the gas containing H₂ from the fractionator 61.

In the second reactor 5, the oligomer reaction and the Fischer-Tropsch reaction take place. In the oligomer reaction, light olefins contained in the first gas react with H₂ to form hydrocarbons having longer carbon chain lengths as exemplified in Formula (7). In the Fischer-Tropsch reaction, the CO and H₂ contained in the third gas react with each other to form CₙH₂ₙ₊₂, that is, saturated hydrocarbons (alkanes) as presented in Formula (8). In this manner, hydrocarbons having a carbon chain length equal to or more than a predetermined length are formed. For example, hydrocarbons are formed in which the number of carbon atoms contained in the carbon chain is six or more. The formed hydrocarbons are liquid hydrocarbons. In addition, a light saturated hydrocarbon gas is generated as a by-product.

The second reactor 5 is connected to the fractionator 51 via a pipe. The second reactor 5 is equipped with a mechanism for discharging the formed hydrocarbons, including liquid hydrocarbons and light saturated hydrocarbon gases. The formed hydrocarbons are discharged from the second reactor 5 and flow through the pipe to the fractionator 51. The reactor for conducting the oligomer reaction and the reactor for conducting the Fischer-Tropsch reaction may be separated from each other. The second reactor 5 may be composed of the reactor for the oligomer reaction and the reactor for the Fischer-Tropsch reaction, which are separated from each other.

The fractionator 51 is configured to fractionate the hydrocarbons formed in the second reactor 5. The fractionator 51 is equipped with a mechanism for introducing the hydrocarbons from the second reactor 5. The fractionator 51 fractionates the introduced hydrocarbons into a light saturated hydrocarbon gas such as CH₄ or C₂H₆, naphtha, and kerosene. The light saturated hydrocarbon gas is a component having the lowest molecular weight among the hydrocarbons formed in the second reactor 5, and is volatile. Kerosene is a component having the highest molecular weight among the formed hydrocarbons and is a liquid hydrocarbon. Among the formed hydrocarbons, naphtha has a higher molecular weight than the light saturated hydrocarbon gas and a lower molecular weight than kerosene. Naphtha is a liquid hydrocarbon and is more volatile than kerosene.

The fractionator 51 is equipped with a mechanism for separately discharging the light saturated hydrocarbon gas, naphtha, and kerosene after fractionation. The fractionated light saturated hydrocarbon gas is discharged from the fractionator 51, passes through the pipe connected to the first reactor 4, and is introduced into the first reactor 4. The fractionator 51 is connected to the heating furnace 52 via a pipe. The fractionated naphtha is discharged from the fractionator 51 and flows through the pipe to the heating furnace 52. A pipe through which the discharged kerosene flows is connected to the fractionator 51. The discharged kerosene is recovered.

The heating furnace 52 is equipped with a mechanism for introducing naphtha from the fractionator 51. The naphtha from the fractionator 51 is introduced into the heating furnace 52. The heating furnace 52 heats the introduced naphtha. The heating furnace 52 heats the naphtha to a temperature sufficient to conduct the reforming reaction. The heating furnace 52 is connected to the reforming reactor 6 via a pipe. The heating furnace 52 is equipped with a mechanism for discharging the heated naphtha. The heated naphtha is discharged from the heating furnace 52 and flows through the pipe to the reforming reactor 6.

The reforming reactor 6 is composed of a fixed bed reactor, and contains a catalyst for the reforming reaction therein. For example, a large number of catalyst carriers carrying catalysts on their surfaces are packed inside the reforming reactor 6. The reforming reactor 6 is equipped with a mechanism for introducing naphtha from the heating furnace 52.

In the reforming reactor 6, the reforming reaction takes place. As presented in Formula (9), dehydrogenation of saturated hydrocarbons contained in naphtha takes place. The reforming reactor 6 is connected to the fractionator 61 via a pipe. The reforming reactor 6 is equipped with a mechanism for discharging the naphtha after being subjected to the reforming reaction. The naphtha after being subjected to the reforming reaction is discharged from the reforming reactor 6 and flows to the fractionator 61.

The fractionator 61 is configured to fractionate naphtha after being subjected to the reforming reaction. The fractionator 61 fractionates naphtha after being subjected to the reforming reaction into H₂ and BTX. The fractionator 61 is equipped with a mechanism for separately discharging H₂ and BTX after fractionation. The fractionated H₂ is discharged from the fractionator 61, passes through the pipe connected to the second reactor 5, and is introduced into the second reactor 5. The discharged BTX is recovered.

As described above in detail, in the present embodiment, the waste plastics are pyrolyzed using a metal oxide as a heat medium to generate the first gas containing H₂, CH₄ and light olefins. Solid carbon formed by the pyrolysis is partially oxidized in a CO₂ atmosphere to generate the second gas containing CO. A steam reforming reaction and a water gas shift reaction are conducted using the second gas and a light saturated hydrocarbon gas to generate the third gas containing CO and H₂. An oligomer reaction and a Fischer-Tropsch reaction are conducted using the first gas and the third gas as raw materials to produce liquid hydrocarbons. As the liquid hydrocarbons, kerosene and BTX are obtained. Kerosene is used as a liquid fuel, so a liquid fuel is produced.

By using a metal oxide as a heat medium, waste plastics are pyrolyzed without performing combustion. Since there is no generation of CO₂ due to combustion, generation of CO₂ decreases compared to a conventional method in which waste plastics are burned. For this reason, by utilizing the formed liquid hydrocarbons, it is possible to suppress the emission of CO₂. For example, by using the formed BTX as an industrial raw material, industrial products can be produced while suppressing the emission of CO₂. For example, a liquid fuel consisting of the formed kerosene can be utilized as an energy source that can suppress the emission of CO₂.

In the present embodiment, as waste plastics are pyrolyzed without being burned, the amount of nitrogen that is mixed into the raw material for liquid hydrocarbons is cut down compared to a method in which waste plastics are burned. In addition, since the solid carbon does not come into direct contact with the air, it is possible to suppress mixing of nitrogen into the raw material for liquid hydrocarbons. For this reason, the amount of nitrogen that is mixed into the raw material for liquid hydrocarbons is small. As the amount of nitrogen mixed into the raw material for liquid hydrocarbons is small, the amount of nitrogen atoms contained in the produced liquid hydrocarbons decreases, and the quality of the liquid hydrocarbons is improved.

In the present embodiment, waste plastics are not completely pyrolyzed into CO and H₂, but the first gas containing CH₄ and light olefins is generated from waste plastics and the first gas is used as a raw material for liquid hydrocarbons. Compared to a conventional method in which waste plastics are completely pyrolyzed into CO and H₂, in the present embodiment, the energy required for pyrolyzing waste plastics decreases. Hence, the energy required to form liquid hydrocarbons from waste plastics decreases, and it is possible to produce a liquid fuel and BTX at a high efficiency.

In the present embodiment, since waste plastics are not completely decomposed into CO and H₂, the amount of oxygen atoms bonded to carbon atoms decreases compared to a conventional method in which waste plastics are burned and decomposed into CO and H₂. The amount of hydrogen atoms consumed to remove oxygen atoms during the formation of hydrocarbons decreases, and the yield of liquid hydrocarbons produced is improved. This makes it possible to produce liquid hydrocarbons at a high efficiency.

### (Experiment)

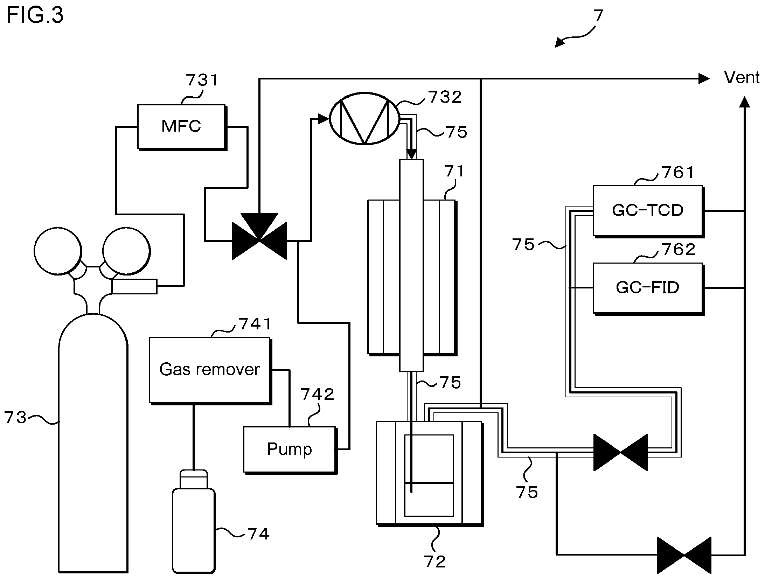

An experiment was conducted to form hydrocarbons by an oligomer reaction and a Fischer-Tropsch reaction in the second reactor 5. FIG. 3 is a block diagram illustrating a configuration example of an experimental apparatus 7 for conducting an experiment to form hydrocarbons by the oligomer reaction and the Fischer-Tropsch reaction. In the figures, the lines connecting the various sections of the experimental apparatus 7 indicate the pipes through which the fluid flows, and the arrows indicate the direction in which the fluid flows. The experimental apparatus 7 includes a reactor 71 corresponding to the second reactor 5. The reactor 71 used in the experiment was a cylindrical reactor having an inner diameter of 8.0 mm and a length of 300 mm. The reactor 71 has an inlet through which raw materials for the reaction flow and an outlet through which products of the reaction flow. The reactor 71 is capable of adjusting the internal temperature and pressure.

The experimental apparatus 7 further includes an ice trap 72, a first tank 73, and a second tank 74. The first tank 73 stores a synthesis gas containing CO, H₂, argon (Ar) and CO₂. The second tank 74 stores 1,5-hexadiene. The ice trap 72 captures products formed by the reaction in the reactor 71.

The first tank 73 is connected to the gas inlet of the reactor 71 via a pipe. The pipe from the first tank 73 to the reactor 71 passes through an MFC (mass flow controller) 731 and a vaporizer 732. The synthesis gas flows from the first tank 73 through the pipe and into the reactor 71. The MFC 731 controls the flow rate of the synthesis gas flowing from the first tank 73 through the pipe. The vaporizer 732 converts the liquid, which has entered therein through the pipe, into a gas.

The pipe connected to the second tank 74 is connected to the pipe, which connects the MFC 731 and the vaporizer 732 to each other, via a gas remover 741 and a pump 742. The 1,5-hexadiene stored in the second tank 74 is liquid, and the gas remover 741 removes the gas contained in the 1,5-hexadiene. The pump 742 pumps 1,5-hexadiene from the second tank 74 and sends the 1,5-hexadiene to the reactor 71. The 1,5-hexadiene is vaporized by the vaporizer 732 and flows into the reactor 71.

The outlet of the reactor 71 is connected to the ice trap 72 via a pipe. The product, which has flowed out of the reactor 71, flows through the pipe into the ice trap 72. Among the components of the product, components having a large molecular weight are captured in the ice trap 72, and components having a small molecular weight flow out from the ice trap 72. The pipe connected to the outlet of the ice trap 72 leads to a vent. A GC-TCD (gas chromatograph - thermal conductivity detector) 761 and a GC-FID (gas chromatograph - flame ionization detector) 762 are connected to a portion of the pipe between the ice trap 72 and the vent. Furthermore, a line heater 75 is provided in the pipe between the vaporizer 732 and the reactor 71, the pipe between the reactor 71 and the ice trap 72, and the pipe between the ice trap 72 and the GC-TCD 761 and GC-FID 762.

Cobalt (Co) was adopted as a raw material for catalytically active species for the oligomer reaction and the Fischer-Tropsch reaction, and a manganese (Mn) compound was adopted as a promoter. Silicon dioxide (SiO₂) and zirconium dioxide (ZrO₂) nano-dispersions were adopted as catalyst carriers. The catalyst carriers carrying catalysts on their surfaces were prepared by impregnation.

In the experiment, 0.5 g of the prepared catalyst carrier carrying a catalyst was packed in the reactor 71. The entire experimental apparatus 7 was purged with hydrogen, and the temperature inside the reactor 71 was set to 400°C and then the temperature was raised under the condition of atmospheric pressure. The catalytic reduction was conducted by allowing H₂ to flow through the reactor 71 at a flow rate of 40 NmL/min for 10 hours.

After the catalytic reduction, the gas flowing through the reactor 71 was changed to the synthesis gas from the first tank 73. The proportions of the respective components in the synthesis gas are as follows: CO 25 vol%, H₂ 65 vol%, Ar 5 vol%, and CO₂ 5 vol%. The flow rate of the synthesis gas was set to 20 NmL/min. In a case where 1,5-hexadiene was added, the pump 742 was operated so that 1,5-hexadiene flowed through the reactor 71 at a predetermined flow rate in addition to the synthesis gas. The predetermined flow rate was set to 3.82 mmol/min. Under two conditions, one in which 1,5-hexadiene was added and the other in which 1,5-hexadiene was not added, the internal pressure of the reactor 71 was set to 1.0 MPaG and the temperature to 240°C, and the reaction for forming hydrocarbons was started in the reactor 71.

The product formed by the reaction in the reactor 71 flows out from the outlet of the reactor 71. The product consists of various kinds of hydrocarbons having different numbers of carbon atoms in the molecule. In the product, gas components having 4 or less carbon atoms, which flowed out from the ice trap 72, were analyzed by the GC-TCD 761 and the GC-FID 762. After 6 hours had elapsed since the start of the reaction, the reaction was stopped by releasing the pressure. In the product, components having 4 or more carbon atoms, which were captured in the ice trap 72, were recovered. The recovered components having 4 or more carbon atoms were separately analyzed by GC-FID.

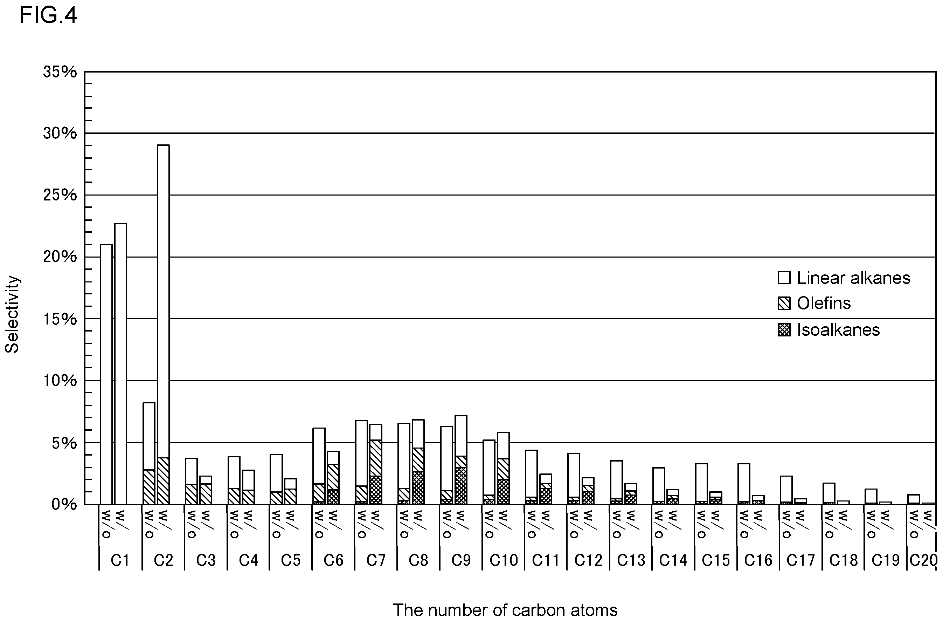

Based on the analytical results, the formed amount of each hydrocarbon contained in the product was calculated. FIG. 4 is a graph illustrating the amount of hydrocarbons formed in the experiment. In FIG. 4, the horizontal axis indicates the number of carbon atoms in a hydrocarbon molecule, and the vertical axis indicates the carbon-based selectivity. In addition, w/o on the horizontal axis indicates that the product was formed under the condition in which 1,5-hexadiene was not added, and w/ indicates that the product was formed under the condition in which 1,5-hexadiene was added. Cn indicates that the number of carbon atoms in a molecule is n. FIG. 4 presents the selectivities of linear alkanes, olefins and isoalkanes formed.

As illustrated in FIG. 4, linear alkanes were mainly obtained as the product under conditions in which 1,5-hexadiene was not added. Under the condition in which 1,5-hexadiene was added, the amounts of olefins and isoalkanes formed increased in addition to linear alkanes. In particular, the isoalkane selectivity of a product having 7 to 15 carbon atoms was significantly improved.

The liquid hydrocarbons according to the present embodiment are produced at a high efficiency using waste plastics as a raw material, and can therefore be utilized as SAF (sustainable aviation fuel). Since the freezing point of liquid fuel is lowered as the liquid fuel contains isoalkanes, the proportion of liquid hydrocarbons having a high isoalkane content mixable in the jet fuel can be increased. The result of improving the isoalkane selectivity in the present embodiment indicates that the method for producing liquid hydrocarbons according to the present embodiment can be one means for producing high-quality SAF.

In the present embodiment, a form in which waste plastics are used as carbon compounds has been described, but the liquid hydrocarbon producing method may be in a form in which carbon compounds other than waste plastics are pyrolyzed to produce liquid hydrocarbons. For example, the liquid hydrocarbon producing method may be in a form in which synthetic resins other than waste plastics are pyrolyzed or a form in which carbon compounds contained in waste materials other than waste plastics are pyrolyzed.

The present invention is not limited to the contents of the embodiments described above, and various modifications can be made within the scope of the claims. In other words, embodiments obtained by combining technical means modified appropriately within the scope of the claims are also included in the technical scope of the present invention.

The features described in the respective embodiments can be combined with each other. In addition, the independent and dependent claims set forth in the claims can be combined with each other in any and all combinations, regardless of the form of reference. Furthermore, the claims are written using in a format in which a claim refers to two or more other claims (multi-claim format), but the present invention is not limited to this format. The claims may be written using a format in which a multi-claim (multi-multi-claim) refers to at least one multi-claim. Reference Signs List

- 1: Pyrolysis furnace
- 2: Partial oxidation furnace
- 3: Chemical looping reactor
- 4: First reactor
- 5: Second reactor
- 6: Reforming reactor
- 7: Experimental apparatus

## Claims

1. A liquid hydrocarbon producing method, **characterized by** comprising:
pyrolyzing a carbon compound using a metal oxide as a heat medium to generate a first gas containing H₂, CH₄, and light olefins;
partially oxidizing solid carbon formed as a by-product on a surface of the metal oxide during the pyrolysis in a CO₂ atmosphere to generate a second gas containing CO;
supplying steam to the second gas and a light saturated hydrocarbon gas and conducting a steam reforming reaction and a water gas shift reaction to generate a third gas containing CO and H₂; and
producing a liquid hydrocarbon using the first gas and the third gas as raw materials.

2. The liquid hydrocarbon producing method according to claim 1, **characterized in that**
a liquid hydrocarbon having a carbon chain length equal to or more than a predetermined length is produced by conducting an oligomer reaction and a Fischer-Tropsch reaction using the first gas and the third gas.

3. The liquid hydrocarbon producing method according to claim 2, **characterized in that**
the light saturated hydrocarbon gas is generated as a by-product when the oligomer reaction or the Fischer-Tropsch reaction is conducted, and
the steam reforming reaction is conducted using the generated light saturated hydrocarbon gas to generate H₂.

4. The liquid hydrocarbon producing method according to claim 1 or 2, **characterized in that**
the solid carbon is reacted with oxygen in the metal oxide in a CO₂ atmosphere to conduct partial oxidation of the solid carbon, and
the metal oxide that is in a reduced state by the partial oxidation is oxidized with oxygen in air to generate heat.

5. The liquid hydrocarbon producing method according to claim 2, **characterized in that**
naphtha and kerosene are formed as the liquid hydrocarbon in the oligomer reaction and the Fischer-Tropsch reaction, and
a reforming reaction is conducted on the formed naphtha to form BTX.

6. The liquid hydrocarbon producing method according to claim 5, **characterized in that**
H₂ generated by the reforming reaction is used to conduct the oligomer reaction and the Fischer-Tropsch reaction.

7. A liquid hydrocarbon producing apparatus, **characterized by** comprising:
a pyrolysis furnace for pyrolyzing a carbon compound using a metal oxide as a heat medium to generate a first gas containing H₂, CH₄, and light olefins;
a partial oxidation furnace for partially oxidizing solid carbon formed as a by-product on a surface of the metal oxide during the pyrolysis in a CO₂ atmosphere to generate a second gas containing CO;
a first reactor for supplying steam to the second gas and a light saturated hydrocarbon gas and conducting a steam reforming reaction and a water gas shift reaction to generate a third gas containing CO and H₂; and
a second reactor for conducting an oligomer reaction of the first gas and a Fischer-Tropsch reaction of the third gas to produce a liquid hydrocarbon having a carbon chain length equal to or more than a predetermined length.
